# EUROPÄISCHE PATENTSCHRIFT

(11) **EP 2 682 607 B1**
(45) Veröffentlichungstag und Bekanntmachung des Hinweises auf die Patenterteilung: **04.03.2015**
(21) Anmeldenummer: 13174673.7
(22) Anmeldetag: 02.07.2013
(51) Int. Cl.: F04B 43/12, F04B 53/22

(54) **Schlauchrollenpumpe mit schwenkbarer Schlauchaufnahme, und medizinisches Gerät für extrakorporale Blutbehandlung**
Hose reel pump with pivotable hose mount, and medical device for extracorporeal blood treatment
Pompe à rouleau tubulaire avec logement de tuyau pivotant et appareil médical pour le traitement extracorporel du sang

(30) Priorität: 03.07.2012 DE 102012105926
(43) Veröffentlichungstag der Anmeldung: 08.01.2014
(73) Patentinhaber: B. Braun Avitum AG, 34212 Melsungen (DE)
(72) Erfinder: Schäfer, Oliver, 36286 Neuenstein (DE); Iske, Andreas, 34320 Söhrewald (DE); Bröker, Björn, 34355 Staufenberg (DE)
(74) Vertreter: Winter, Brandl, Fürniss, Hübner, Röss, Kaiser, Polte - Partnerschaft mbB

(56) Entgegenhaltungen:
- EP-A2- 1 241 355
- JP-A- 2008 000 425
- US-A- 3 963 023
- US-A1- 2012 082 576
- US-B2- 8 047 819

## Beschreibung

Die Erfindung betrifft ein Schlauch rollen pumpe für ein medizinisches Gerät zur extrakorporalen Blutbehandlung mit einem Pumpengehäuse, das eine gebogene Lauffläche und einen innerhalb der Lauffläche drehbaren Rotor aufweist, wobei ein Schlauchsegment schlaufenförmig zwischen die Lauffläche und den Rotor einbringbar ist.

Die Erfindung betrifft ferner ein medizinisches Gerät zur extrakorporalen Blutbehandlung mit einer solchen Schlauchrollenpumpe.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt.

Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird. Derartige Überleitsysteme werden vorzugsweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird. Um die Handhabung während dieses Ablegens und Aufrüstens des Überleitsystems zu erleichtern, ist es ferner bekannt, an beiden Enden des Schlauchsegments jeweils einen Konnektor vorzusehen, der mit einer zu- bzw. abführenden Leitung verbunden werden kann.

Zur Aufnahme eines Schlauchs innerhalb einer Rollenpumpe beschreibt beispielsweise das Patent US 8,047,819 B2 Haltevorrichtungen, die lösbar am Pumpengehäuse angebracht sind. So können für verschiedene Schlauchgrößen und -arten unterschiedliche Haltevorrichtungen an der Pumpe montiert werden. Eine Haltevorrichtung weist dabei eine Klemmvorrichtung mit wenigstens einer schwenkbaren Klemmbacke auf, die eine halbkreisförmige Ausnehmung aufweist, so dass ein Schlauch in dieser halbkreisförmigen Ausnehmung und einer gegenüber liegenden, ebenfalls halbkreisförmigen Ausnehmung einer anderen Klemmbacke gehalten werden kann. Die Klemmbacken können auch mehrere dieser Ausnehmungen aufweisen, so dass mehrere Schläuche gleichzeitig aufgenommen werden können.

Darüber hinaus sind automatische Systeme bekannt, die das Ein- und Ausfädeln des Schlauchs in die Pumpe übernehmen und somit erleichtern sollen. Oftmals ist dabei zum Ausfädeln ein Aktuator zu betätigen, der das System beispielsweise über einen Linearantrieb aus seiner Therapieposition in eine Ausfädelposition bewegt. Hierzu kann es bei solchen Systemen erforderlich sein, einen Schalter/Knopf an dem medizinischen Gerät zu bedienen bzw. einen Softwarebutton auf einer Benutzeroberfläche zu berühren.

Ferner sind Multikonnektoren bekannt, die beide Anschlüsse für zu- bzw. abführende Leitungen in einem Bauteil vereinigen, das dann in eine Aufnahme des Pumpengehäuses eingebracht werden kann. Über die geometrische Form derartiger Multikonnektoren kann auch ihre Präsenz in der Pumpe detektiert werden, indem beispielsweise ein zylindrischer Abschnitt des Multikonnektors während des Einsetzvorgangs einen Stößel betätigt, dessen axiale Position über eine Lichtschranke abgefragt wird. Gleichzeitig ist dieser Stößel Bestandteil eines im Pumpengehäuse montierten elektromechanischen Aktuators, der den Multikonnektor über einen Linearantrieb auswerfen kann.

Um ein Schlauchsegment an einer Schlauchrollenpumpe jeweils in die Ein- und Ausfädelposition zu bewegen, sind beispielsweise aus der JP 2008-000425 mehrere Varianten eines Schwenkbauteils bzw. Kipphebels bekannt, an dem beide Enden des schlaufenförmigen Schlauchsegments angebracht werden können. In einer ersten Schwenkposition des Kipphebels befindet sich das Schlauchsegment dann in einer Lage, in welcher der Einfädelvorgang gestartet werden kann, während das Schlauchsegment in einer anderen Schwenklage ausgefädelt werden kann. Der automatische Ein- und Ausfädelvorgang erfolgt durch Führungsstifte am Umfang des Rotors, welche den Schlauch bei Drehung des Rotors in das Pumpengehäuse hinein- oder aus ihm herausdrücken.

Die Enden des schlaufenförmigen Schlauchsegments werden beide durch den Kipphebel angehoben bzw. abgesenkt, so dass die Führungsstifte entsprechend ausgeformt sein müssen, um den Schlauch in diesen beiden Lagen erfassen und in eine bestimmte Richtung drücken zu können. Dabei muss der automatische Ein- und Ausfädelvorgang verlässlich durchführbar sein, der Schlauch darf dabei jedoch nicht zu stark deformiert werden.

US 3963023 zeigt eine Schlauchrollenpumpe für ein medizinisches Gerät zur extrakorporalen Blutbehandlung mit einem Pumpengehäuse, das eine gebogene Lauffläche und einen innerhalb der Lauffläche drehbaren Rotor aufweist, wobei ein Schlauchsegment schlaufenförmig zwischen die Lauffläche und den Rotor einbringbar ist.

Am Pumpengehäuse ist ein Kipphebel mit einer (zweiten) Aufnahme zur Aufnahme des (anderen) Endes des schlaufenförmigen Schlauchsegments vorgesehen sind, wobei der Kipphebel so um eine Schwenkachse drehbar ist, dass die (zweite) Aufnahme mit dem Schlauchsegment in eine Einfädelposition bringbar ist. Der Kipphebel ist in verschiedene Lagen bringbar.

Eine dezidierte Ausfädelposition ist nicht gezeigt.

Zum Ausfädeln wird im Betrieb der Pumpe der Hebel angehoben woraufhin sofort ein Ausfädelprozess stattfindet.

Ausgehend hiervon ist es die Aufgabe der Erfindung, eine Schlauchrollenpumpe für ein medizinisches Gerät zur extrakorporalen Blutbehandlung bereitzustellen, welche eine Anbringung eines Schlauchsegments ermöglicht, bei der das Schlauchsegment automatisiert in die Schlauchrollenpumpe ein- und ausfädelbar ist.

Aufgabe der Erfindung ist es ferner, ein medizinisches Gerät zur extrakorporalen Blutbehandlung mit einer solchen Schlauchrollenpumpe bereitzustellen.

Erfindungsgemäß wird diese Aufgabe durch eine Schlauchrollenpumpe gemäß dem unabhängigen Anspruch 1 gelöst. Vorteilhafte Weiterbildungen der Schlauchrollenpumpe ergeben sich aus den Unteransprüchen 2-14. Die Aufgabe wird ferner durch ein medizinisches Gerät zur extrakorporalen Blutbehandlung gemäß Anspruch 15 gelöst.

Die erfindungsgemäße Schlauchrollenpumpe eignet sich für ein medizinisches Gerät zur extrakorporalen Blutbehandlung und weist ein Pumpengehäuse auf, das eine gebogene Lauffläche und einen innerhalb der Lauffläche drehbaren Rotor aufweist, wobei ein Schlauchsegment schlaufenförmig zwischen die Lauffläche und den Rotor einbringbar ist. Erfindungsgemäß sind am Pumpengehäuse eine fest stehende erste Aufnahme zur Aufnahme eines Endes des schlaufenförmigen Schlauchsegments und ein Kipphebel mit einer zweiten Aufnahme zur Aufnahme des anderen Endes des schlaufenförmigen Schlauchsegments vorgesehen, wobei der Kipphebel so um eine Schwenkachse drehbar ist, dass die zweite Aufnahme mit dem Schlauchsegment in wenigstens zwei verschiedene Lagen bringbar ist, und dass eine erste Lage eine Einfädelposition und eine zweite Lage eine Ausfädelposition für das Schlauchsegment darstellt.

Durch diese Ausgestaltung der Schlauchrollenpumpe kann ein Schlauchsegment auf einfache Weise an einem Pumpengehäuse angebracht werden. Dabei kann ein Ende eines schlaufenförmigen Schlauchsegments in eine Aufnahme am Pumpengehäuse eingebracht werden, wobei diese Aufnahme feststeht und das Schlauchsegment somit in diesem Bereich am Pumpengehäuse fixiert ist. Das andere Ende des schlaufenförmigen Schlauchsegments wird dagegen in die Aufnahme am Kipphebel eingebracht, so dass das Schlauchsegment in diesem Bereich beweglich ist. Hierdurch kann dieser Bereich des Schlauchsegments sowohl in eine Einfädel- als auch in eine Ausfädelposition gebracht werden.

Vorzugsweise sind die Aufnahmen als als hinterschnittene Ausnehmungen ausgebildet, in die das elastische Schlauchsegment eindrückbar ist. Nach der Therapie kann der Schlauch wieder aus den Aufnahmen herausgezogen werden.

Ferner können am Rotor Mittel zum automatisierten Ein- und Ausfädeln des Schlauchsegments zwischen den Rotor und die Lauffläche vorgesehen sein. Hierbei ist beispielsweise am Umfang des Rotors wenigstens ein Führungsstift angebracht, der beim Drehen des Rotors Kontakt mit dem Schlauch aufnimmt und ihn in eine bestimmte Richtung drückt. Die zweite Aufnahme befindet sich dann in der Ausfädelposition in einer Lage, in welcher sich der wenigstens eine Führungsstift bei einer Rotation des Rotors zwischen dem Schlauchsegment und dem Boden des Pumpengehäuses bewegt und das Schlauchsegment hierdurch aus dem Pumpengehäuse herausheben kann. Durch Schwenken des Kipphebels von dem Pumpengehäuse weg wird die zweite Aufnahme und damit das Schlauchsegment somit soweit angehoben, dass der wenigstens eine Führungsstift das Schlauchsegment ausfädeln kann.

Im Gegensatz dazu befindet sich die zweite Aufnahme in der Einfädelposition in einer Lage, in welcher sich der wenigstens eine Führungsstift bei einer Rotation des Rotors oberhalb des Schlauchsegments bewegt und das Schlauchsegment hierdurch in das Pumpengehäuse hineindrückbar ist. Durch Schwenken des Kipphebels wird das Schlauchsegment somit über oder unter den wenigstens einen Führungsstift bewegt, damit dieser den Einfädel- oder Ausfädelvorgang durchführen kann.

Der Vorteil der feststehenden ersten Aufnahme hat dabei insbesondere den Vorteil, dass diese Aufnahme nicht angehoben oder abgesenkt wird, sondern so an dem Pumpengehäuse fixiert ist, dass es in diesem Bereich zu keinen Einquetschungen des Schlauchs kommt. Hierdurch wird das Risiko eine Hämolyse während der Therapie verringert, so dass es insbesondere beim Einfädelvorgang vor der Therapie wichtig ist, dass der Schlauch nicht ungünstig verformt wird. Daher handelt es sich bei der feststehenden ersten Aufnahme vorzugsweise um diejenige Aufnahme, in deren Richtung der Schlauch beim Einfädeln durch den wenigstens einen Führungsstift leicht geschoben wird.

In einem Ausführungsbeispiel der Erfindung sind an der Schlauchrollenpumpe Mittel zum Halten des Kipphebels in der Einfädelposition vorgesehen. Dazu können beispielsweise abseits der Schwenkachse Mittel zum Verrasten des Kipphebels mit dem Pumpengehäuse vorgesehen sein, so dass der Kipphebel und damit die zweite Aufnahme mit dem Schlauchsegment manuell von einem Bediener in die Einfädelposition geschwenkt werden können und dort von selbst gehalten werden. Hierzu kann am Pumpengehäuse eine Rastnase ausgebildet sein, die zusammen mit der Geometrie des Kipphebels einen Rastmechanismus bildet, wobei die Verrastung im Bereich der zweiten Aufnahme erfolgt.

Darüber hinaus sind vorzugsweise auch Mittel zum Halten des Kipphebels in der Ausfädelposition vorgesehen. Auch diese können als Rastmechanismus realisiert werden, wobei am Pumpengehäuse eine Rastnase ausgebildet ist, die zusammen mit einer Rastfeder am Kipphebel einen Rastmechanismus bildet. Diese Rastfeder kann gebogen ausgeführt sein und im Bereich der Schwenkachse am Kipphebel angebracht sein, wobei die Biegung der Rastfeder im Wesentlichen um die Schwenkachse herum verläuft.

Um die jeweilige Position des Kipphebels in den verschiedenen Lagen detektieren zu können, kann die Geometrie und/oder das Material des Kipphebels so ausgestaltet sein, dass der Kipphebel wenigstens in der Einfädelposition und der Ausfädelposition direkt durch Detektionsmittel an der Schlauchrollenpumpe detektierbar ist. Sobald diese Positionen eindeutig detektiert wurden, kann der automatisierte Einfädel- bzw. Ausfädelvorgang initiiert werden.

Dabei können verschiedene Detektionsmechanismen zur Anwendung kommen. Beispielsweise ist am Kipphebel wenigstens ein Magnet und/oder ein ferromagnetisches Material angebracht. Der Magnet kann beispielsweise von dem Kunststoff umspritzt sein, aus dem der Kipphebel besteht, oder es handelt sich um einen plastizifierten Magneten. Durch eine magnetisch wirkende Sensorik beispielsweise mit Hall-Sensoren an verschiedenen Positionen können dann verschiedene Stellungen des Kipphebels detektiert werden. In dieser Ausführungsform der Erfindung erfolgt die Erkennung somit wenigstens über die Materialeigenschaften des Kipphebels, da ein magnetischer Bereich vorhanden sein muss, aber auch die Geometrie des Bauteils ist entscheidend, um die verschiedenen Stellungen des Kipphebels sicher erkennen zu können. Dazu muss der Magnet in einem bestimmten Bereich angebracht sein, wobei dieser Bereich besonders ausgeformt sein kann, um ihn durch die Sensorik erfassen zu können.

Das gilt ebenso für eine Ausführungsform eines Kipphebels, bei dem am Grundkörper und/oder den Konnektorelementen wenigstens ein ferromagnetisches Material angebracht ist. Auch das ferromagnetische Material kann beispielsweise von Kunststoff umspritzt sein. Hierdurch ist es möglich, verschiedene Stellungen des Kipphebels durch eine induktiv wirkende Sensorik zu erfassen, wobei die Detektion anhand des Geometrie und der Materialeigenschaften erfolgt.

Die Detektion der verschiedenen Positionen kann auch optisch erfolgen. In einem weiteren Ausführungsbeispiel der Erfindung ist der Kipphebel beispielsweise in wenigstens einem Bereich lichtundurchlässig oder lichtzerstreuend, und angrenzend an diesen Bereich oder innerhalb dieses Bereichs ist ein lichtdurchlässiger Ausbruch vorgesehen. Dabei kann der Kipphebel außerhalb des lichtundurchlässigen oder lichtzerstreuenden Bereichs transparent sein, oder der gesamte Kipphebel ist als lichtundurchlässiger oder lichtzerstreuender Bereich ausgeführt. In dieser Ausführungsform der Erfindung erfolgt die Erkennung der Kipphebelposition über die Geometrie und die Materialeigenschaften des Kipphebels, wobei diese Eigenschaften für eine optische Detektion genutzt werden.

Bei einer solchen Ausgestaltung eines Kipphebels ist es dann beispielsweise möglich, über wenigstens eine Lichtschranke zu ermitteln, in welcher Stellung sich der Kipphebel gerade befindet, denn das Licht einer Lichtschranke wird entweder unterbrochen, wenn es auf einen lichtundurchlässigen oder lichtzerstreuenden Bereich trifft, oder kann den Grundkörper im Bereich des Ausbruchs durchdringen. Ein wenigstens teilweise transparenter Kipphebel hat dabei den Vorteil, dass Licht nicht nur durch ihn hindurch geleitet werden kann, um von einer Lichtschranke detektiert zu werden. Vielmehr kann die Position der Sendedioden der Lichtschranke hierdurch auch freier gewählt werden, denn es besteht die Möglichkeit, dass das Licht einer Lichtschranke erst einen transparenten Teilbereich des Kipphebels durchdringt, bevor es auf einen Ausbruch oder einen lichtundurchlässigen oder lichtzerstreuenden Bereich trifft. Dies erleichtert die Gestaltung der Sensorik am Pumpengehäuse.

In einem weiteren Ausführungsbeispiel des Kipphebels bilden die Geometrie und das Material des Kipphebels einen Lichtleiter, durch den Licht entlang eines definierten Weges durch den Kipphebel leitbar ist. Vorzugsweise ist der Lichtleiter dabei so ausgebildet, dass Licht von der Schwenkachse durch den Kipphebel zur zweiten Aufnahme leitbar ist. Durch eine entsprechende Anordnung von wenigstens einer Sendediode, die Licht in den Kipphebel einkoppelt, und wenigstens einer Empfangsdiode, die Licht aus dem Kipphebel empfängt, können so mittels einer Sensorik verschiedene Stellungen des Kipphebel s erkannt werden, indem die Dioden entsprechend in diesen verschiedenen Stellungen angeordnet werden.

Von der Erfindung umfasst ist ferner ein medizinisches Gerät zur extrakorporalen Blutbehandlung, umfassend wenigstens eine Schlauchrollenpumpe mit einem Pumpengehäuse, das eine gebogene Lauffläche und einen innerhalb der Lauffläche drehenden Rotor aufweist, wobei ein Schlauchsegment eines extrakorporalen Blutkreislaufs zwischen die Lauffläche und den Rotor einbringbar ist, und dem Schlauchsegment Blut über eine zuführende Leitung zuführbar ist, während Blut von dem Schlauchsegment über eine abführende Leitung abführbar ist. Erfindungsgemäß umfasst das Gerät eine Schlauchrollenpumpe gemäß einer der beschriebenen Ausführungsformen.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Aufsicht auf eine Schlauchrollenpumpe mit eingelegtem Schlauchsegment;
- Fig. 3: eine schematische Seitenansicht einer Schlauchrollenpumpe nach Fig. 2 beim Einlegen eines Schlauchsegments;
- Fig. 4: eine schematische Seitenansicht einer Schlauchrollenpumpe nach Fig. 3 beim Eindrücken des Kipphebels in den Rastmechanismus;
- Fig. 5: eine schematische Seitenansicht einer Schlauchrollenpumpe nach Fig. 3 mit geschlossenem Deckel in der Einfädelposition;
- Fig. 6: eine schematische Seitenansicht einer Schlauchrollenpumpe nach Fig. 3 mit geschlossenem Deckel in der Ausfädelposition;
- Fig. 7a: eine vergrößerte Ansicht des Rastmechanismus im Bereich der Schwenkachse in der Ausfädelposition; und
- Fig. 7b: eine vergrößerte Ansicht des Rastmechanismus im Bereich der Schwenkachse in der Einfädelposition.

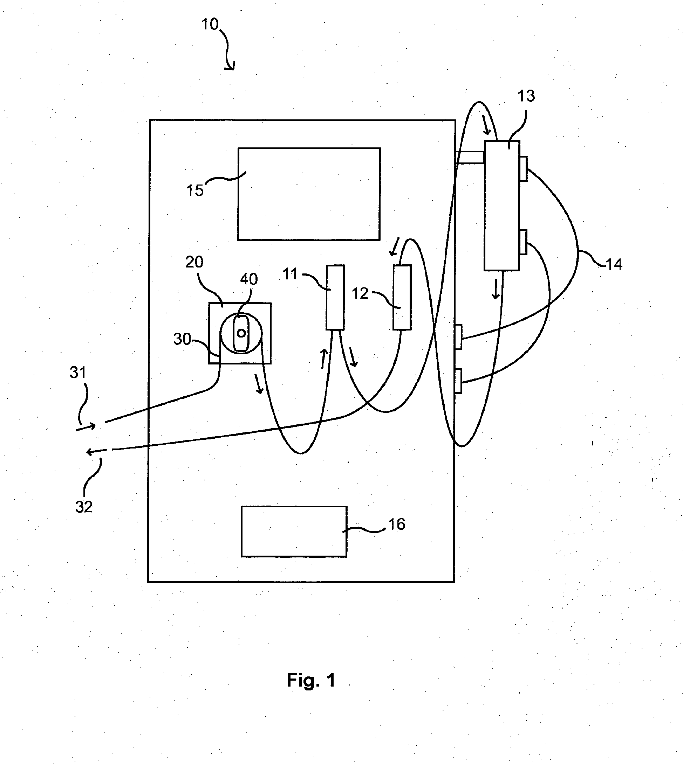

Fig. 1 zeigt eine schematische Darstellung der wesentlichen Grundkomponenten eines medizinischen Geräts 10 zur extrakorporalen Blutbehandlung mit einer Blutpumpe, wobei es sich bei der Blutpumpe um eine Schlauchrollenpumpe handelt. Die Schlauchrollenpumpe weist dabei ein Pumpengehäuse 20 auf, das typischerweise an der Frontseite des Dialysegeräts 10 angebracht ist.

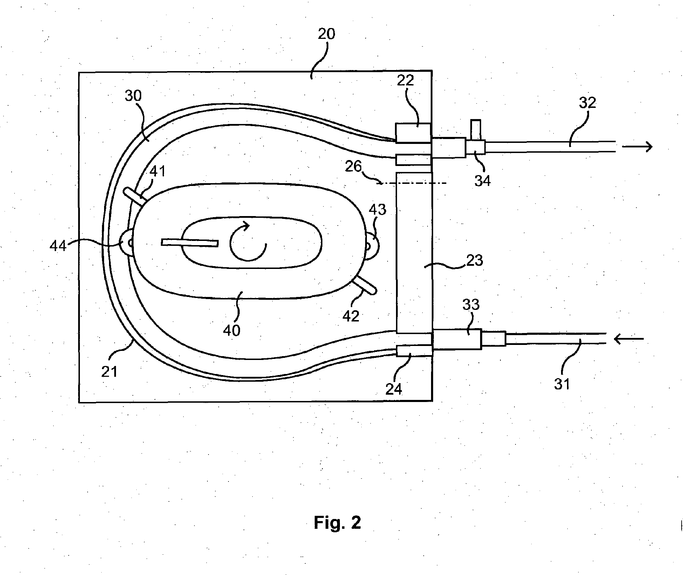

Dieser Schlauchrollenpumpe wird arterielles Blut 31 eines Patienten zugeführt und durch den extrakorporalen Blutkreislauf gefördert. Anschließend wird das Blut als venöses Blut 32 wieder zum Patienten zurückgeführt. Dabei wird das Blut mittels der Pumpe durch ein Überleitsystem gefördert, das an mehrere Komponenten des Dialysegeräts 10 angeschlossen ist, wobei ein Schlauchsegment 30 des Überleitsystems in die Blutpumpe eingelegt ist und ein Rotor 40 das Blut peristaltisch durch dieses Schlauchsegment 30 fördert, wie es einer vergrößerten Ansicht der Fig. 2 zu entnehmen ist.

Nach Durchlaufen der Blutpumpe gelangt das Blut zum Dialysator 13, nachdem es vorzugsweise zuvor einen arteriellen Luftfänger 11 durchlaufen hat. Im Dialysator 13 wird das Blut durch Stoffaustausch mit einem Dialysat 14 gereinigt, welches dem Dialysator 13 zu- und abgeführt wird. Nach Durchlaufen des Dialysators 13 gelangt das Blut zu einem venösen Luftfänger 12 und wird anschließend dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 15 erfolgen, die vorzugsweise als Touchscreen ausgebildet ist. Ferner weist das Dialysegerät 13 eine Steuer-/Regeleinheit 16 auf.

Fig. 2 stellt eine schematische Darstellung einer Schlauchrollenpumpe mit eingelegtem Schlauchsegment 30 dar. Die Schlauchrollenpumpe weist das Pumpengehäuse 20 auf, das für den Bediener des Geräts leicht zugänglich, wobei es mit einem (in Fig. 2 nicht dargestellten) Deckel 50 abdeckbar ist, der über ein Scharnier zum Beispiel nach oben oder zur Seite schwenkbar ist, um Zugriff auf die Pumpe zu erhalten.

In dem Pumpengehäuse 20 ist durch eine Vertiefung im Gehäuse eine kurvenförmige Lauffläche 21 ausgebildet, in welche das Schlauchsegment 30 schlaufenförmig eingelegt werden kann, so dass seine beiden Schlauchenden aus dem Gehäuse 20 herausragen. Dabei kann die Vertiefung mit einer Seitenfläche in dem Pumpengehäuse 20 ausgebildet sein, die im Wesentlichen gleichmäßig senkrecht zur Frontseite des Geräts verläuft, oder die Lauffläche 21 ist ungleichmäßig durch eine Seitenfläche der Vertiefung ausgeformt, die konkav oder sogar in sich verdreht ausgeformt ist.

Innerhalb der Lauffläche 21 ist ein Rotor 40 angebracht, der einen elliptischen Umfang hat, so dass er das Schlauchsegment 30 bei der Rotation an seinen Hauptscheiteln punktuell zusammendrücken kann. An diesen Hauptscheiteln oder anderen Bereichen sind hierzu Druckrollen 43 und 44 angebracht. Durch die Drehung des Rotors 40 im Uhrzeigersinn bewegt sich der Bereich eines zusammengedrückten Schlauchsegments 30 ebenfalls im Uhrzeigersinn, bis sich der zugehörige Hauptscheitel bzw. die Druckrolle 44 wieder vom Schlauchsegment 30 löst. In der Zeit hat der gegenüber liegende Hauptscheitel bzw. die Druckrolle 43 jedoch bereits wieder Kontakt zu dem Schlauchsegment 30 aufgenommen, so dass Blut jeweils in dem Bereich des Schlauchsegments 30, vor dem es von dem Rotor 40 zusammendrückt wird, peristaltisch vom Pumpeneingang zum Pumpenausgang gefördert wird. Der Rotor kann neben einem elliptischen Umfang jedoch auch jegliche andere geeignete Form haben.

Das Schlauchsegment 30 ist über Verbindungselemente 33 und 34 mit einer zuführenden Leitung 31 und einer abführenden Leitung 32 verbunden, wie es bei Standard-Überleitsystemen der Fall ist. Ein Ende des Schlauchsegments 30 ist in eine erste Aufnahme 22 eingebracht, die im oberen Bereich des Pumpengehäuses 20 ausgebildet ist. Diese Aufnahme 22 ist fest in das Pumpengehäuse 20 integriert und als hinterschnittene Ausnehmung ausgebildet" so dass das Schlauchsegment 30 in diesen Längsschlitz eingedrückt wurde. Sie kann jedoch aus als mit dem Pumpengehäuse 20 fest verbundene, längsgeschlitzte Hülse ausgestaltet sein.

Unterhalb der oberen Aufnahme 22 ist ein Kipphebel 23 gelenkig angebracht, der um eine Schwenkachse 26 drehbar ist. Am schwenkbaren Ende des Kipphebels 23 ist eine zweite Aufnahme 24 ausgebildet, die ebenfalls als hinterschnittene Ausnehmung ausgebildet ist, in die das andere Ende des Schlauchsegments 30 eingedrückt werden kann. Dabei liegen die Verbindungselemente 33 und 34 zum Verbinden der zu- und abführenden Leitungen 31, 32 mit dem jeweiligen Ende des Schlauchsegments 30 außen an den Aufnahmen 22 und 24 an, so dass die zu- und abführenden Leitungen 31, 32 nicht in die Pumpe hineingezogen werden können.

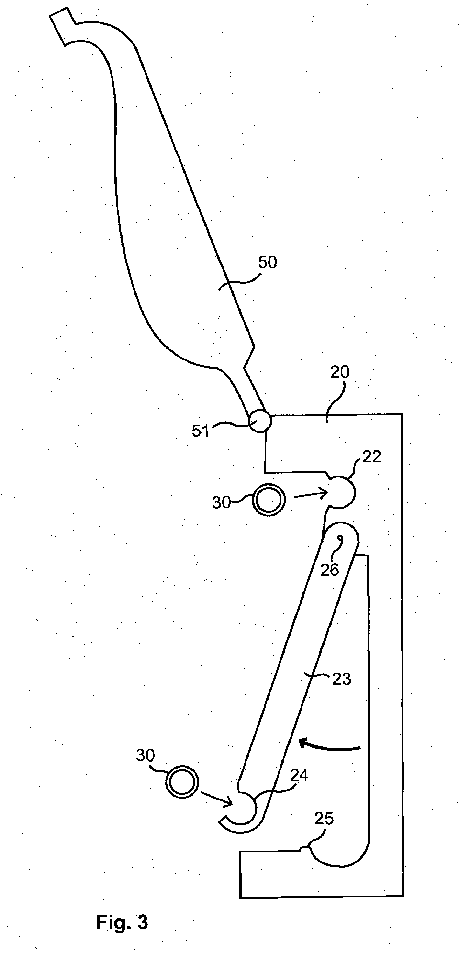

Fig. 3 zeigt eine schematische Seitenansicht der Schlauchrollenpumpe mit geöffnetem Deckel 50, der zum Einlegen eines Schlauchsegments 30 um ein Scharnier 51 nach oben geschwenkt wurde. Bei dem Deckel 50 kann es sich jedoch auch um einen seitlich verschwenkbaren Deckel 50 handeln. Der Kipphebel 23 ist um die Schwenkachse 26 herausgeschwenkt bzw. in der in der Fig. 3 gezeigten Ansicht nach geschwenkt.

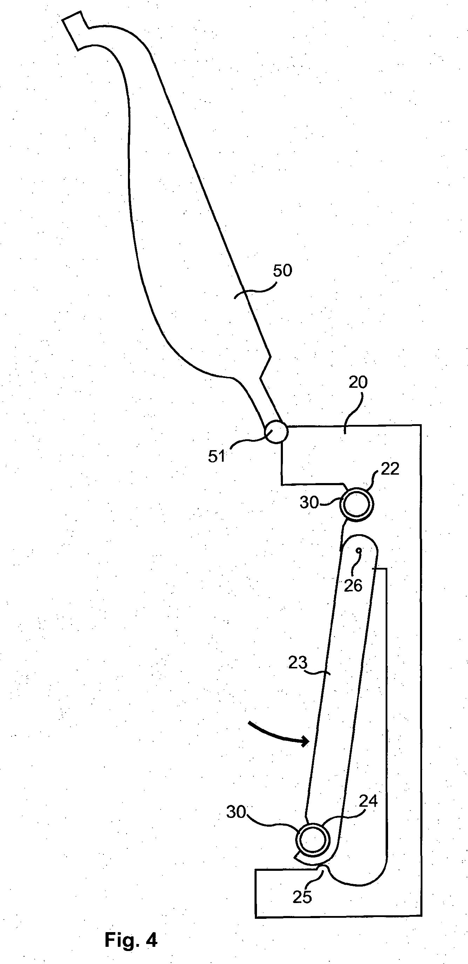

Das eine Ende des Schlauchsegments 30 wird in die obere Aufnahme 22 gedrückt, wobei diese Bewegung in Fig. 3 durch einen Pfeil dargestellt ist. Das Gleiche gilt für das andere Ende des Schlauchsegments 30, das in die untere Aufnahme 24 eingedrückt wird. Anschließend wird der Kipphebel 23 zusammen mit dem daran angebrachten Schlauchsegment 30 in Richtung Boden der Vertiefung im Pumpengehäuse 20 gedrückt, um den Kipphebel 23 in die Einfädelposition zu bringen (nach rechts in der Fig. 4). Damit der Kipphebel 23 in der Einfädelposition gehalten wird, ist im unteren Bereich des Kipphebels 23 ein Rastmechanismus ausgebildet. Hierzu ist beispielsweise eine Rastnasengeometrie 25 vorgesehen, über welche der untere Bereich des Kipphebels 23 gedrückt werden muss, bevor er in einer dahinter liegenden Aufnahme des Pumpengehäuses 20 einrastet.

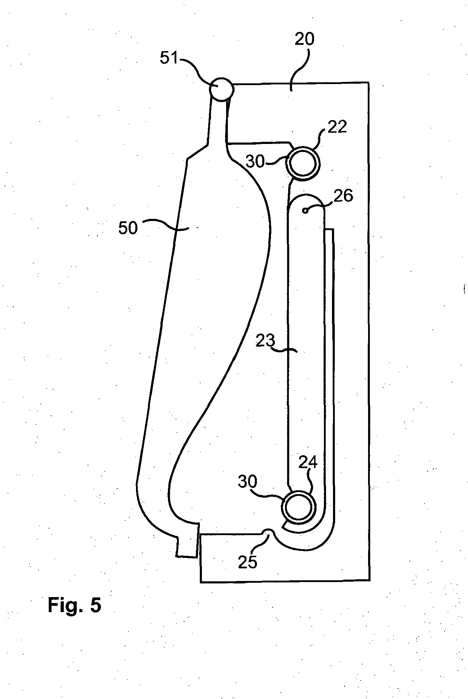

Vorzugsweise sind (nicht dargestellte) Detektionsmittel vorgesehen, um die Lage des Kipphebels 23 in der eingerasteten Einfädelposition zu detektieren. Diese Einfädelposition stellt gleichzeitig die Therapieposition dar. Nach Einrasten des Kipphebels 23 in dieser Position wird der Deckel 50 geschlossen und der automatisierte Einfädelvorgang kann gestartet werden. Vorzugsweise sind ebenfalls Detektionsmittel zum Erkennen des Zustands des Deckels 50 vorgesehen, so dass der Einfädelvorgang gestartet werden kann, sobald über die Detektionsmittel sichergestellt ist, dass sich der Kipphebel 23 in der Einfädelposition befindet und der Deckel 50 geschlossen ist (Fig. 5).

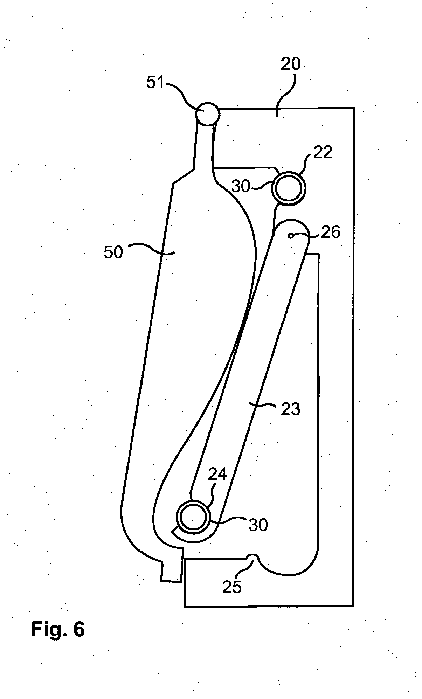

Beim Einfädelvorgang wird der Rotor 40 langsam gedreht, so dass die Führungsstifte 41 und 42 den Einfädelvorgang durchführen. Dabei bewegt sich wenigstens ein Führungsstift 41 bzw. 42 außen entlang des Schlauchsegments 30 und drückt diesen zwischen den Rotor 40 und die Lauffläche 21. Um nach der Therapie den Ausfädelvorgang zu initiieren, wird der Kipphebel 23 von einem Benutzer zusammen mit dem Schlauchsegment 30 nach links gezogen, wie es in Fig. 6 gezeigt ist. Dabei dient der Deckel 50 als Gegenlager bzw. Anschlag, gegen den der Kipphebel 23 bis zum Anschlag gezogen werden kann. Dieser Anschlag definiert die Ausfädelposition, die ebenfalls durch Detektionsmittel erkannt werden kann. Bei geschlossenem Deckel 50 und dem Kipphebel 23 in der Ausfädelposition kann der Rotor 40 die Führungsstifte langsam zwischen dem Pumpenboden und dem Schlauchsegment 30 bewegen, wodurch das Schlauchsegment 30 zwischen dem Rotor 40 und der Lauffläche 21 herausgehoben wird.

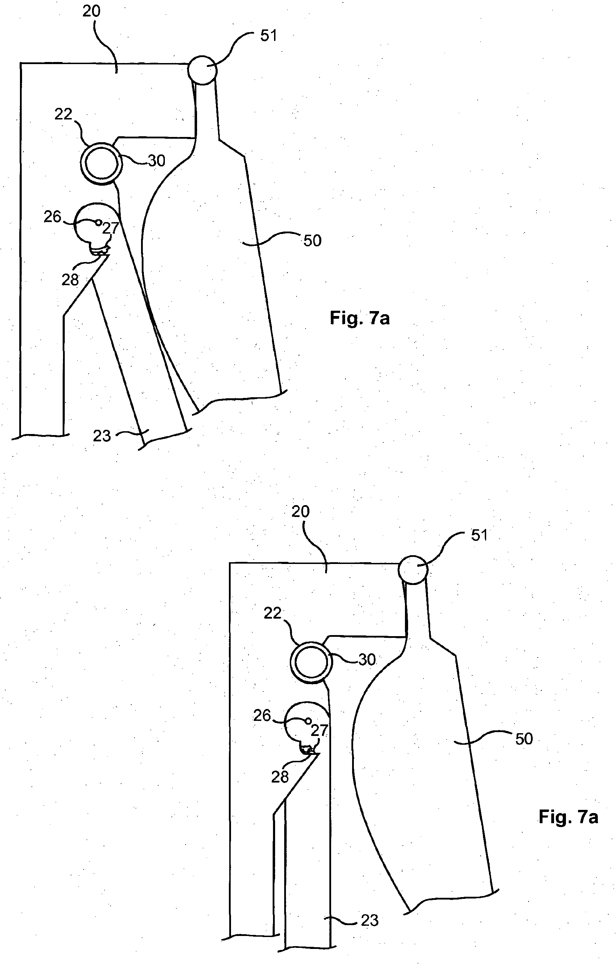

Damit der Kipphebel 23 in der Ausfädelposition gehalten werden kann, ist beispielsweise im Bereich der Schwenkachse 26 ein Federmechanismus vorgesehen. Die Funktionsweise dieses Federmechanismus ist den Figuren 7a und 7b zu entnehmen, die einen vergrößerten Ausschnitt des Drehpunkts darstellen. In der Fig. 7a ist der Kipphebel 23 in der Ausfädelposition gezeigt, wobei auf dieser Seite des Kipphebels 23 ein bogenförmiges Federelement 27 an dem Kipphebel 23 angebracht ist. Dieses Federelement 27 verläuft im Wesentlichen um die Schwenkachse 26 herum und weist ein Rastelement in Form einer Erhöhung auf. Diese Erhöhung stößt in dieser Stellung des Kipphebel 23 an eine Rastnase 28 an, die an dem Pumpengehäuse 20 ausgeformt ist. Der Kipphebel 23 ist somit in der Ausfädelposition an der Schwenkachse 26 verrastet und rutscht bzw. schwenkt alleine durch das Gewicht des Kipphebels 23 nicht wieder zurück.

Beim Einschwenken des Kipphebel 23 aus der Ausfädelstellung in die Einfädelposition muss dieser nicht nur in die untere Verrastung (Rastnase 25) gedrückt werden, sondern muss auch die Verrastung im Bereich der Schwenkachse 26 überwunden werden. Dies ist in Fig. 7b dargestellt, wobei ersichtlich ist, dass das Federelement 27 beim Schwenken um den Drehpunkt über die Rastnase 28 am Pumpengehäuse 20 gezogen wurde.

### Bezugszeichenliste

- 10: Medizinisches Gerät zur extrakorporalen Blutbehandlung, Dialysegerät
- 11: Arterieller Luftfänger
- 12: Venöser Luftfänger
- 13: Dialysator
- 14: Dialysat
- 15: Anzeige-/Eingabeeinheit, Touchscreen
- 16: Steuer-/Regelungseinheit
- 20: Pumpengehäuse
- 21: Lauffläche
- 22: Aufnahme, feststehend
- 23: Kipphebel
- 24: Aufnahme, beweglich
- 25: Rastgeometrie
- 26: Schwenkachse
- 27: Rastfeder
- 28: Rastnase
- 30: Pumpensegment, Schlauchsegment
- 31: Leitung, zuführend, arterielles Blut vom Patienten
- 32: Leitung, abführend, Venöses Blut zum Patienten
- 33,34: Konnektor
- 40: Rotor
- 41,42: Führungsstift
- 43,44: Druckrolle
- 50: Deckel, Pumpendeckel
- 51: Scharnier

## Patentansprüche

1. Schlauchrollenpumpe für ein medizinisches Gerät zur extrakorporalen Blutbehandlung mit einem Pumpengehäuse (20), das eine gebogene Lauffläche (21) und einen innerhalb der Lauffläche (21) drehbaren Rotor (40) aufweist, wobei ein Schlauchsegment (30) schlaufenförmig zwischen die Lauffläche (21) und den Rotor (40) einbringbar ist,
wobei
am Pumpengehäuse (20) eine fest stehende erste Aufnahme (22) zur Aufnahme eines Endes des schlaufenförmigen Schlauchsegments (30) und ein Kipphebel (23) mit einer zweiten Aufnahme (24) zur Aufnahme des anderen Endes des schlaufenförmigen Schlauchsegments (30) vorgesehen sind, und
der Kipphebel (23) so um eine Schwenkachse (26) drehbar ist, dass die zweite Aufnahme (24) mit dem Schlauchsegment (30) in wenigstens zwei verschiedene Lagen bringbar ist, und
eine erste Lage eine Einfädelposition und eine zweite Lage eine Ausfädelposition für das Schlauchsegment (30) darstellt.

2. Schlauchrollenpumpe nach Anspruch 1,
**dadurch gekennzeichnet, dass** die Aufnahmen (22, 24) als hinterschnittene Ausnehmungen ausgebildet sind, in die das jeweilige Ende des elastischen Schlauchsegments (30) eindrückbar ist.

3. Schlauchrollenpumpe nach einem oder beiden der Ansprüche 1 und 2,
**dadurch gekennzeichnet, dass** am Rotor (40) Mittel zum automatisierten Ein- und Ausfädeln des Schlauchsegments (30) zwischen den Rotor (40) und die Lauffläche (21) vorgesehen sind.

4. Schlauchrollenpumpe nach Anspruch 3,
**dadurch gekennzeichnet, dass** am Umfang des Rotors (40) wenigstens ein, insbesondere radial verspringender, Führungsstift (41, 42) angebracht ist.

5. Schlauchrollenpumpe nach Anspruch 4,
**dadurch gekennzeichnet, dass** sich die zweite Aufnahme (24) in der Ausfädelposition in einer Lage befindet, in welcher sich der wenigstens eine Führungsstift (41, 42) bei einer Rotation des Rotors (40) zwischen dem Schlauchsegment (30) und dem Boden des Pumpengehäuses (20) bewegt und das Schlauchsegment (30) hierdurch aus dem Pumpengehäuse (20) heraushebt.

6. Schlauchrollenpumpe nach einem oder beiden der Ansprüche 4 und 5,
**dadurch gekennzeichnet, dass** sich die zweite Aufnahme (24) in der Einfädelposition in einer Lage befindet, in welcher sich der wenigstens eine Führungsstift (41, 42) bei einer Rotation des Rotors (40) oberhalb des Schlauchsegments (30) bewegt und das Schlauchsegment (30) hierdurch in das Pumpengehäuse (20) hineindrückt.

7. Schlauchrollenpumpe nach einem oder mehreren der Ansprüche 1 bis 6,
**dadurch gekennzeichnet, dass** Mittel zum Halten des Kipphebels (23) in der Einfädelposition vorgesehen sind.

8. Schlauchrollenpumpe nach Anspruch 7,
**dadurch gekennzeichnet, dass** von der Schwenkachse (26) Mittel zum Verrasten des Kipphebels (23) mit dem Pumpengehäuse (20) vorgesehen sind.

9. Schlauchrollenpumpe nach Anspruch 8,
**dadurch gekennzeichnet, dass** am Pumpengehäuse (20) eine Rastnase (25) ausgebildet ist, die zusammen mit der Geometrie des Kipphebels (23) einen Rastmechanismus bildet, wobei die Verrastung im Bereich der zweiten Aufnahme (24) erfolgt.

10. Schlauchrollenpumpe nach einem oder mehreren der Ansprüche 1 bis 9,
**dadurch gekennzeichnet, dass** Mittel zum Halten des Kipphebels (23) in der Ausfädelposition vorgesehen sind.

11. Schlauchrollenpumpe nach Anspruch 10,
**dadurch gekennzeichnet, dass** am Pumpengehäuse (20) eine Rastnase (28) ausgebildet ist, die zusammen mit einer Rastfeder (27) am Kipphebel (23) einen Rastmechanismus bildet.

12. Schlauchrollenpumpe nach Anspruch 11,
**dadurch gekennzeichnet, dass** die Rastfeder (27) gebogen und im Bereich der Schwenkachse (26) am Kipphebel (23) angebracht ist, wobei die Biegung der Rastfeder (27) im Wesentlichen um die Schwenkachse (26) herum verläuft.

13. Schlauchrollenpumpe nach einem oder mehreren der Ansprüche 1 bis 12,
**dadurch gekennzeichnet, dass** die Geometrie und/oder das Material des Kipphebels (23) so ausgestaltet sind, dass der Kipphebel (23) wenigstens in der Einfädelposition und/oder der Ausfädelposition direkt durch Detektionsmittel an der Schlauchrollenpumpe detektierbar ist.

14. Schlauchrollenpumpe nach Anspruch 13,
**dadurch gekennzeichnet, dass** am Kipphebel (23) wenigstens ein Magnet und/oder ein ferromagnetisches Material angebracht ist und die Detektionsmittel wenigstens einen Hallsensor aufweisen.

15. Medizinisches Gerät zur extrakorporalen Blutbehandlung, umfassend wenigstens eine Schlauchrollenpumpe mit einem Pumpengehäuse (20), das eine gebogene Lauffläche (21) und einen innerhalb der Lauffläche (21) drehenden Rotor (40) aufweist, wobei ein Schlauchsegment (30) eines extrakorporalen Blutkreislauf zwischen die Lauffläche (21) und den Rotor (40) einbringbar ist, und dem Schlauchsegment (30) Blut über eine zuführende Leitung (31) zuführbar ist, während Blut von dem Schlauchsegment (30) über eine abführende Leitung (32) abführbar ist,
**dadurch gekennzeichnet, dass** das Gerät eine Schlauchrollenpumpe gemäß einem oder mehreren der Ansprüche 1 bis 14 umfasst.

## Claims

1. A tube roller pump for a medical device for extracorporeal blood treatment comprising a pump housing (20) having a bent bearing surface (21) and a rotor (40) rotatable inside the bearing surface (21), wherein a tubing segment (30) can be introduced between the bearing surface (21) and the rotor (40) in the form of a loop,
wherein
a stationary first receiving portion (22) for accommodating one end of the loop-shaped tubing segment (30) and a rocker arm (23) including a second receiving portion (24) for accommodating the other end of the loop-shaped tubing segment (30) are provided at the pump housing (20), and
the rocker arm (23) is rotatable about a pivoting axis (26) so that the second receiving portion (24) with the tubing segment (30) can be brought into at least two different positions, and
a first position constitutes a threading position and a second position constitutes an unthreading position for the tubing segment (30).

2. The tube roller pump according to claim 1,
**characterized in that** the receiving portions (22, 24) are in the form of undercut recesses into which the respective end of the elastic tubing segment (30) can be pressed.

3. The tube roller pump according to one or both of claims 1 and 2,
**characterized in that** means for automated threading and unthreading of the tubing segment (30) between the rotor (40) and the bearing surface (21) are provided at the rotor (40).

4. The tube roller pump according to claim 3,
**characterized in that** at least one especially radially projecting guide pin (41, 42) is disposed at the periphery of the rotor (40).

5. The tube roller pump according to claim 4,
**characterized in that** in the unthreading position the second receiving portion (24) is at a position at which the at least one guide pin (41, 42) moves upon rotation of the rotor (40) between the tubing segment (30) and the bottom of the pump housing (20) and thereby lifts the tubing segment (30) out of the pump housing (20).

6. The tube roller pump according to one or both of claims 4 and 5,
**characterized in that** in the threading position the second receiving portion (24) is at a position at which the at least one guide pin (41, 42) moves upon rotation of the rotor (40) above the tubing segment (30) and thereby presses the tubing segment (30) into the pump housing (20).

7. The tube roller pump according to one or more of claims 1 to 6,
**characterized in that** means for maintaining the rocker arm (23) are provided in the threading position.

8. The tube roller pump according to claim 7,
**characterized in that** means for locking the rocker arm (23) with the pump housing (20) are provided spaced apart from the pivoting axis (26).

9. The tube roller pump according to claim 8,
**characterized in that** at the pump housing (20) a stop catch (25) is formed which forms a stop mechanism together with the geometry of the rocker arm (23), the locking being effected in the area of the second receiving portion (24).

10. The tube roller pump according to one or more of claims 1 to 9,
**characterized in that** means for maintaining the rocker arm (23) are provided in the unthreading position.

11. The tube roller pump according to claim 10,
**characterized in that** a stop catch (28) forming a stop mechanism together with a stop spring (27) at the rocker arm (23) is formed at the pump housing (20).

12. The tube roller pump according to claim 11,
**characterized in that** the stop spring (27) is bent and arranged in the area of the pivoting axis (26) at the rocker arm (23), the bending of the stop spring (27) substantially extending around the pivoting axis (26).

13. The tube roller pump according to one or more of claims 1 to 12,
**characterized in that** the geometry and/or the material of the rocker arm (23) are configured so that the rocker arm (23) can be detected at least in the threading position and/or the unthreading position directly by detecting means at the tube roller pump.

14. The tube roller pump according to claim 13,
**characterized in that** at least a magnet and/or a ferromagnetic material is/are arranged at the rocker arm (23) and the detecting means include at least a Hall sensor.

15. A medical device for extracorporeal blood treatment comprising at least a tube roller pump including a pump housing (20) having a bent bearing surface (21) and a rotor (40) rotating inside the bearing surface (21), wherein a tubing segment (30) of an extracorporeal blood circulation can be introduced between the bearing surface (21) and the rotor (40) and blood can be fed to the tubing segment (30) via a feeding line (31), while blood can be discharged from the tubing segment (30) via a discharging line (32),
**characterized in that** the device comprises a tube roller pump according to one or more of claims 1 to 14.

## Revendications

1. Pompe à rouleau tubulaire pour un appareil médical pour le traitement extracorporel du sang, comportant un boîtier de pompe (20) qui présente une surface de roulement (21) courbe et un rotor (40) pouvant tourner dans la surface de roulement (21), dans laquelle un segment de tuyau (30) peut être inséré entre la surface de roulement (21) et le rotor (40) en formant une boucle,
dans laquelle
sur le boîtier de pompe (20), sont prévus un premier logement fixe (22) pour la réception d'une extrémité du segment de tuyau (30) formant une boucle et un culbuteur (23) comportant un deuxième logement (24) pour la réception de l'autre extrémité du segment de tuyau (30) formant une boucle, et
le culbuteur (23) peut tourner autour d'un axe de pivotement (26) de telle sorte que le deuxième logement (24) puisse être placé avec le segment de tuyau (30) dans au moins deux situations différentes, et
une première situation représente une position d'insertion et une deuxième situation représente une position de retrait pour le segment de tuyau (30).

2. Pompe à rouleau tubulaire selon la revendication 1,
**caractérisée en ce que** les logements (22, 24) sont formés en tant qu'évidements de contre-dépouille dans lesquels l'extrémité respective du segment de tuyau (30) élastique peut être enfoncée.

3. Pompe à rouleau tubulaire selon l'une ou les deux des revendications 1 et 2,
**caractérisée en ce que**, sur le rotor (40) sont prévus des moyens d'insertion et de retrait automatisés du segment de tuyau (30) entre le rotor (40) et la surface de roulement (21).

4. Pompe à rouleau tubulaire selon la revendication 3,
**caractérisée en ce que**, sur le pourtour du rotor, est aménagée au moins une broche de guidage (41, 42) en particulier décalée radialement.

5. Pompe à rouleau tubulaire selon la revendication 4,
**caractérisée en ce que** le deuxième logement (24) dans la position de retrait se trouve dans une situation dans laquelle l'au moins une broche de guidage (41, 42) est mobile par une rotation du rotor (40) entre le segment de tuyau (30) et le fond du boîtier de pompe (20) et le segment de tuyau (30) se soulève au travers de celui-ci hors du boîtier de pompe (20).

6. Pompe à rouleau tubulaire selon une ou les deux des revendications 4 et 5,
**caractérisée en ce que** le deuxième logement (24) dans la position d'insertion se trouve dans une situation dans laquelle l'au moins une broche de guidage (41, 42) est mobile par une rotation du rotor (40) au-dessus du segment de tuyau (30) et le segment de tuyau (30) appuie au travers de celui-ci dans le boîtier de pompe (20).

7. Pompe à rouleau tubulaire selon une ou plusieurs des revendications 1 à 6,
**caractérisée en ce que** des moyens de retenue du culbuteur (23) sont prévus dans la position d'insertion.

8. Pompe à rouleau tubulaire selon la revendication 7,
**caractérisée en ce que**, à partir de l'axe de pivotement (26), des moyens d'encliquetage du culbuteur (23) sont prévus avec le boîtier de pompe (20).

9. Pompe à rouleau tubulaire selon la revendication 8,
**caractérisée en ce que**, sur le boîtier de pompe (20), est formé un bec d'encliquetage (25) qui forme conjointement avec la géométrie du culbuteur (23) un mécanisme d'encliquetage, dans lequel l'encliquetage s'effectue dans la zone du deuxième logement (24).

10. Pompe à rouleau tubulaire selon une ou plusieurs des revendications 1 à 9,
**caractérisée en ce que** des moyens de retenue du culbuteur (23) sont prévus dans la position de retrait.

11. Pompe à rouleau tubulaire selon la revendication 10,
**caractérisée en ce que** sur le boîtier de pompe (20) est formé un bec d'encliquetage (28) qui forme, conjointement avec un ressort d'encliquetage (27), sur le culbuteur (23), un mécanisme d'encliquetage.

12. Pompe à rouleau tubulaire selon la revendication 11,
**caractérisée en ce que** le ressort d'encliquetage (27) est courbe et est monté dans la zone de l'axe de pivotement (26) sur le culbuteur (23), dans laquelle la courbure du ressort d'encliquetage (27) évolue essentiellement autour de l'axe de pivotement (26).

13. Pompe à rouleau tubulaire selon une ou plusieurs des revendications 1 à 12,
**caractérisée en ce que** la géométrie et/ou le matériau du culbuteur (23) est conçu(e) de telle sorte que le culbuteur (23) puisse être détecté au moins dans la position d'insertion et/ou dans la position de retrait directement par un moyen de détection sur la pompe à rouleau tubulaire.

14. Pompe à rouleau tubulaire selon la revendication 13,
**caractérisée en ce que**, sur le culbuteur (23), est monté au moins un aimant et/ou un matériau ferromagnétique et les moyens de détection présentent au moins un capteur Hall.

15. Appareil médical pour le traitement extracorporel du sang, comprenant au moins une pompe à rouleau tubulaire comportant un boîtier de pompe (20) qui présente une surface de roulement courbe (21) et un rotor (40) tournant dans la surface de roulement (21), dans laquelle un segment de tuyau (30) d'une circulation sanguine extracorporelle peut être inséré entre la surface de roulement (21) et le rotor (40), et du sang peut être acheminé au segment de tuyau (30) par une conduite d'acheminement (31) alors que du sang peut être évacué du segment de tuyau (30) par une conduite d'évacuation (32),
**caractérisé en ce que** l'appareil comprend une pompe à rouleau tubulaire selon une ou plusieurs des revendications 1 à 14.
